# FASCICULE DE BREVET EUROPEEN

(11) **EP 2 097 131 B1**
(45) Date de publication et mention de la délivrance du brevet: **18.04.2012**
(21) Numéro de dépôt: 03789507.5
(22) Date de dépôt: 26.11.2003
(51) Int. Cl.: A61N 1/05

(54) **SYSTEME A ELECTRODE PRET-A-MONTER POUR CARDIOVERSION ET ENSEMBLE DUDIT SYSTEME ET D'UN ENDOSCOPE**
ELEKTRODENSYSTEM BAUSATZ UND ENTSPRECHENDE ANORDNUNG DIESES SYSTEMS MIT EINEM ENDOSKOP
READY-TO-INSTALL ELECTRODE SYSTEM FOR CARDIOVERSION AND ASSEMBLY OF SAID SYSTEM AND AN ENDOSCOPE

(30) Priorité: 27.11.2002 FR 0214842
(43) Date de publication de la demande: 09.09.2009
(73) Titulaire: Benhalima, Bouziane, 93500 Pantin (FR); Benhalima, Zohra, 93500 Pantin (FR)
(72) Inventeur: Benhalima, Bouziane, 93500 Pantin (FR); Benhalima, Zohra, 93500 Pantin (FR)
(74) Mandataire: Laget, Jean-Loup
(86) Numéro de dépôt international: PCT/FR2003/003495
(87) Numéro de publication internationale: WO 2004/050174

(56) Documents cités:
- EP-A- 0 104 287
- WO-A-90/13259
- WO-A-98/18519
- WO-A1-01/52740
- US-A- 4 149 538
- US-A- 5 178 149
- US-B1- 6 394 949

## Description

La présente invention concerne un système à électrode prêt-à-monter destiné à être utilisé avec un endoscope pour effectuer une cardioversion par voie oesop hagienne, et un ensemble dudit système et d'un endoscope destiné à être utilisé pour effectuer une cardioversion par voie oesophagienne.

Pour traiter des troubles du rythme chez un patient, et en particulier une fibrillation auriculaire, il est connu de procéder à une cardioversion par chocs électriques. Une telle cardioversion peut conduire à des accidents vasculaires cérébraux ou à des embolies périphériques, par détachement de tout ou partie d'un thrombus préexistant au niveau de l'auricule gauche. Ainsi, une échographie transoesophagienne est donc généralement réalisée préalablement à la cardioversion, afin de visualiser correctement l'auricule gauche et ainsi détecter la présence éventuelle d'un thrombus. Une sonde connue pour une telle échographie transoesophagienne comporte généralement un capteur à ultrasons à l'extrémité distale d'un endoscope.

Une méthode de cardioversion utilisée à ce jour consiste à produire des chocs électriques par voie interne ou endocavitaire. Une telle méthode constitue un acte invasif très lourd à mettre en oeuvre. Une autre méthode consiste à produire les chocs électriques par voie externe en appliquant deux électrodes sur le thorax du patient. Cette méthode est relativement simple à mettre en oeuvre mais nécessite une anesthésie générale.

Une autre méthode de cardioversion consiste à produire des chocs électriques par voie oesophagienne à l'aide d'un endoscope muni à sa portion distale de plusieurs électrodes. Cette opération nécessite une simple sédation mais peut entraîner des complications lorsqu'elle suit l'échographie transoesophagienne préalable. En effet, dans la pratique, l'opérateur éprouve parfois des difficultés à réintroduire une nouvelle sonde dans la bouche du patient où elle peut s'enrouler car il existe un risque de rejet de la sonde par le patient du fait que les tissus oesophagiens sont déjà irrités, sans compter les risques de lésion ou de perforation de l'oesophage par suite des introductions répétées des endoscopes. Pour éviter de telles complications, le demandeur a proposé, dans la demande WO 98/18519, un endoscope muni d'un capteur à ultrasons et d'au moins une électrode à son extrémité distale qui permet d'effectuer à la fois une échographie et une cardioversion transoesophagienne lors d'une même opération, soit simultanément, soit successivement, sans nécessiter plusieurs introductions et retraits de sondes à travers l'oesophage. En disposant une électrode sur la partie articulée de l'endoscope à proximité du capteur à ultrasons, un tel endoscope permet en outre à l'opérateur de visualiser exactement l'endroit où il veut pratiquer la cardioversion et d'assurer un rapprochement étroit de l'électrode contre la paroi du coeur, conduisant ainsi à une cardioversion particulièrement efficace.

Par ailleurs, pour des raisons d'hygiène et de sécurité de la santé publique, il est préférable de protéger l'extrémité distale des endoscopes qui est introduite dans l'oesophage, pour chaque opération, au moyen d'une housse de protection à usage unique en latex ou polyuréthane. L'utilisation d'une telle housse de protection se révèle incompatible avec la présence d'électrodes intégrées à l'extrémité distale d'un endoscope, car l'énergie électrique libérée localement par les électrodes est susceptible de faire fondre ou brûler la fine paroi de la housse. Une éventuelle solution qui aurait pu être envisagée consisterait à prévoir une housse en matériau ayant une résistance thermique et une épaisseur suffisantes pour résister à l'énergie électrique libérée par les électrodes. Toutefois une telle solution entraînerait un coût rédhibitoire étant donné l'usage unique de la housse de protection.

Par conséquent, afin d'opérer dans des conditions d'hygiène et de sécurité plus sûres, les médecins préfèrent actuellement utiliser la cardioversion avec chocs électriques par voie externe, malgré ses inconvénients.

Le but de la présente invention est de proposer un système à électrode permettant de réaliser une cardioversion par endoscope qui soit simple à mettre en oeuvre, efficace dans le traitement des troubles du rythme, bien supportée par le patient et qui soit plus sûre en terme d'hygiène et de sécurité de la santé publique.

Les documents US 6 394 949 et WO 01/52740 décrivent des systèmes correspondant au préambule de la revendication 1.

A cet effet, la présente invention a pour objet un système à électrode prêt-à-monter destiné à être utilisé avec un endoscope pour effectuer une cardioversion par voie oesophagienne, comportant une housse de protection de forme générale tubulaire présentant une extrémité distale fermée et une extrémité proximale ouverte, ladite housse étant de stinée à être emmanchée sur l'endoscope pour recouvrir au moins l'extrémité distale de l'endoscope, au moins une électrode comprenant une membrane électrique conductrice connectée à un fil de conduction électrique destiné à être relié par son extrémité libre à un appareil de cardioversion, des premiers moyens de fixation aptes à permettre la fixation de ladite au moins une électrode sur l'extrémité distale de l'endoscope, et des seconds moyens de fixation aptes à permettre la fixation de ladite au moins une électrode sur la paroi tubulaire de la housse, de façon que ladite membrane conductrice soit directement accessible de l'extérieur de la housse, caractérisé par le fait que

le système selon l'invention comprend au moins une bande support apte à être fixée par une première face dite interne sur l'endoscope par lesdits premiers moyens de fixation et portant sur sa seconde face dite externe ladite au moins une électrode, la paroi tubulaire de ladite housse de protection étant munie d'au moins une ouverture de dimensions inférieures à celles de la bande support, les seconds moyens de fixation étant prévus sur la housse de protection et/ou la bande support pour permettre une fixation de la bande support par sa seconde face externe contre la face interne de la paroi tubulaire de la housse de protection, de sorte que ladite au moins une ouverture soit fermée et que la membrane conductrice de ladite électrode soit accessible de l'extérieur à travers ladite ouverture.

Selon un mode de réalisation, la ou les électrodes font corps avec la housse de protection, par exemple par moulage des électrodes dans la paroi de la housse, les seconds moyens de fixation étant constitués par le corps même de la housse, le(s) fils de conduction pouvant être incorporés dans la paroi de la housse ou être disposés le long de la face interne de la paroi tubulaire de la housse.

Selon une particularité, la largeur de l'ouverture de la housse de protection est au moins égale à la dimension transversale de la membrane conductrice de l'électrode, de sorte que ladite membrane conductrice soit logée à travers ladite ouverture lorsque la bande support est fixée à la housse de protection par les seconds moyens de fixation.

Selon une autre particularité, la surface de la première face interne de la bande support comprend une première couche auto-adhésive qui constitue lesdits premiers moyens de fixation.

Selon une autre particularité, les seconds moyens de fixation sont constitués par une deuxième couche auto-adhésive située à la périphérie de l'ouverture sur la face interne de la paroi tubulaire de la housse de protection et/ou sur la périphérie de la seconde face externe de la bande support.

Avantageusement, chaque couche auto-adhésive est recouverte d'une pellicule pelable destinée à être retirée avant la fixation de la bande support.

Le système selon l'invention peut comprendre avantageusement de 3 à 6 électrodes espacées longitudinalement sur la bande support, la paroi tubulaire de la housse de protection étant munie d'une ouverture longitudinale de longueur correspondante.

Dans un mode de réalisation, le ou les fils de conduction sont portés par la bande support, s'étendent jusqu'au bord transversal proximal de la bande support et sont logés à partir de ce bord transversal proximal dans une gaine isolante reliée à son extrémité opposée à un connecteur permettant la connexion du ou des fils de conduction à l'appareil de cardioversion.

Avantageusement, la ou les électrodes sont recouvertes par au moins un film de protection apte à être retiré avant l'utilisation de la ou des électrodes.

Le système selon l'invention, comprenant la ou les électrodes et la housse de protection, permet un usage unique. L'utilisation d'une telle housse avec des électrodes selon l'invention permet de proposer une housse à usage unique et à faible coût par rapport à une solution envisageable qui consisterait à prévoir une housse, recouvrant un endoscope muni d'électrodes, en un matériau apte à résister à l'énergie électrique libérée par les électrodes. Par ailleurs, la couverture d'électrodes intégrées à l'extrémité d'un endoscope par une housse en latex ou polyuréthane rend inefficace la cardioversion en raison de la nature non-conductrice d'une telle housse. Ainsi, une éventuelle solution qui aurait pu être envisagée consisterait à prévoir une housse munie de zones conductrices destinées à venir en vis -à-vis des électrodes. La housse selon l'invention présente également un coût nettement plus faible que celui d'une telle housse avec des zones conductrices.

Selon une autre particularité, le système selon l'invention comporte en outre un moyen de commande externe comprenant un boîtier muni de moyens de montage pour permettre le montage dudit boîtier sur l'extrémité proximale de l'endoscope, d'un premier moyen de connexion externe, tel qu'une fiche réceptrice externe, pour connecter le ou les fils de conduction de la ou des électrodes, en particulier le connecteur de la gaine défini précédemment au boîtier, d'un deuxième moyen de connexion pour connecter le boîtier à l'appareil de cardioversion, et d'un module pour la charge de l'énergie électrique destinée à produire les chocs électriques et pour le déclenchement de ces chocs, ledit module étant commandé par un organe de commande externe, tel qu'un bouton, et étant relié aux premier et second moyens de connexion.

La présente invention a également pour objet un ensemble du système tel que défini précédemment et d'un endoscope muni à son extrémité proximale d'une poignée de commande, destiné à être utilisé pour effectuer une cardioversion par voie oesophagienne, ledit ensemble se caractérise par le fait que la housse de protection est emmanchée sur l'endoscope et recouvre au moins l'extrémité distale de l'endoscope, ladite au moins une électrode est fixée d'une part sur l'extrémité distale de l'endoscope par les premiers moyens de fixation et, d'autre part, sur la paroi tubulaire de la housse par les seconds moyens de fixation de façon que ladite membrane conductrice soit directement accessible de l'extérieur de la housse, ledit fil de conduction étant relié par son extrémité libre à un premier moyen de connexion externe situé au niveau de ladite poignée de commande.

Dans une première variante, la poignée de commande intègre un moyen de commande interne de cardioversion, ledit système comprenant un module pour la charge de l'énergie électrique destinée à produire les chocs électriques et pour le déclenchement de ces chocs, ledit module étant commandé par un organe externe de commande et étant relié audit premier moyen de connexi on, tel qu'une fiche réceptrice externe, pour connecter la ou les fils de conduction de la ou des électrodes, en particulier le connecteur de la gaine défini précédemment, et à un deuxième moyen de connexion pour le connecter à l'appareil de cardioversion.

Dans une seconde variante, l'ensemble comprend un moyen de commande externe comprenant un boîtier muni de moyens de montage pour permettre le montage dudit boîtier sur l'extrémité proximale de l'endoscope, dudit premier moyen de connexion externe, tel qu'une fiche réceptrice externe, pour connecter le ou les fils de conduction de la ou des électrodes au boîtier, d'un deuxième moyen de connexion pour connecter le boîtier à l'appareil de cardioversion, et d'un module pour la charge de l'énergie électrique de stinée à produire les chocs électriques et pour le déclenchement de ces chocs, ledit module étant commandé par un organe de commande externe et étant relié aux premier et second moyens de connexion.

Avantageusement, une portion distale de l'endoscope est munie d'un capteur à ultrasons de manière à former un dispositif permettant d'effectuer à la fois une échographie et une cardioversion par voie oesophagienne. Avantageusement, la portion distale de l'endoscope qui comporte le capteur est articulée par rapport au reste de l'endoscope, au moins une électrode étant fixée de part et d'autre de l'articulation.

L'ensemble selon l'invention peut être monté en réalisant les étapes suivantes : fixation de la bande support par sa première face interne sur l'extrémité distale de l'endoscope, fixation de la bande support par sa deuxième face externe sur la paroi interne de la housse de protection, de sorte que la ou les électrodes soient disposées au niveau d'une ouverture de ladite housse de protection, et connexion de la ou des électrodes à un moyen de commande de cardioversion relié à un appareil de cardioversion. Avantageusement, l'étape de fixation de la bande support sur l'extrémité distale de l'endoscope est réalisée avant l'étape de fixation de la bande support su r la housse de protection.

L'invention sera mieux comprise, et d'autres buts, détails, caractéristiques et avantages apparaîtront plus clairement au cours de la description explicative détaillée qui va suivre de deux modes de réalisation particuliers actuellement préférés de l'invention, donnés uniquement à titre illustratif et non limitatif, en référence au dessin schématique annexé.

Sur ce dessin:
- la figure 1 représente une vue de dessus d'une bande support portant des électrodes d'un système à électrode prêt- à-monter selon l'invention ;
- la figure 2 représente une vue très schématique agrandie partielle et en coupe de la bande support de la figure 1, selon le plan II-II;
- la figure 3 est une vue partielle externe de l'ensemble selon l'invention ;
- la figure 4 est une vue partielle en coupe de la figure 3 selon le plan IV-IV ;
- la figure 5 est une vue schématique partielle d'une poignée de commande de cardioversion selon une première variante ; et,
- la figure 6 est une vue schématique et en plan d'un système de commande de cardioversion selon une deuxième variante.

Suivant l'exemple de réalisation représenté sur les figures, le système à électrode selon l'invention comprend une bande support 1 portant des électrodes 2a-d et une housse de protection 3 séparée.

La bande support 1, de forme rectangulaire possède une première face 11, dite interne, présentant une surface entièrement adhésive. Cette face interne 11 est entièrement recouverte d'une pellicule pelable 4 destinée à être retirée avant l'appl ication de la bande support sur l'extrémité distale d'un endoscope, tel que décrit ci-après. La bande support est réalisée en un matériau flexible non conducteur. En variante, il pourrait être prévu une face interne dont la surface présente une ou plusieurs couches adhésives permettant d'assurer une bonne fixation de la bande support sur l'endoscope. Quatre électrodes 2a- d identiques sont disposées longitudinalement sur la deuxième face 12 de la bande, dite externe, opposée à la face inférieure 11. Chaque électrode comprend, de manière connue, une membrane conductrice circulaire 21 supportée par une membrane protectrice 22 non conductrice, par exemple de forme circulaire, par laquelle l'électrode est fixée par collage sur la face externe 12 de la bande support, la membrane conductrice étant sensiblement disposée parallèlement à la bande support 1. La membrane protectrice 21 recouvre le pourtour circulaire de la membrane conductrice de manière à protéger l'oesophage contre les brûlures locales liées aux décharges électriques lors de la cardioversion, en particulier en cas d'utilisation d'un niveau d'énergie important. Les membranes conductrices 21 des électrodes sont chacune reliées à un fil de conduction 5a- d. Chaque fil de conduction 5a-d est soudé par une extrémité à la membrane conductrice 21 d'une électrode, s'étend perpendiculairement à la membrane conductrice depuis cette extrémité soudée à travers la membrane de protection 22, traverse la bande support 1 et s'étend longitudinalement, le long de la face inférieure 11, en direction d'un des bords transversaux de la bande, dit bord proximal 13. Les fils de conduction, espacés transversalement les uns des autres, adhèrent à la surface adhésive de la face inférieure 12 de la bande support et sont pris en sandwich entre ladite face inférieure et la pellicule pelable 4. Au niveau du bord proximal 13, les fils de conduction sont disposés dans une gaine isolante 6. La gaine isolante est fixée par une extrémité au bord proximal 13 et se termine à son autre extrémité par un connecteur 7 pour permettre de connecter électriquement les quatre fils de conduction à un moyen de commande de cardioversion décrit ci -après.

Dans une variante de réalisation, il pourrait être prévu de disposer les fils de conduction sur la face externe de la bande support, les fils de conduction étant alors maintenus espacés les uns des autres contre la face externe par des bandes adhésives disposées entre les électrodes, ou de les disposer entre deux couches d'une bande support composite.

La face externe 12 de la bande support présente une surface comportant une couche adhésive 17 périphérique qui comprend deux portions longitudinales et deux portions transversales, formées respectivement le long des bords longitudinaux 15, 16 et transversaux 13, 14 de la bande support. Cette couche adhésive 17 est recouverte d'une pellicule pelable 8 constituée de quatre portions, deux d'entre elles recouvrant la couche adhésive 17 le long des bords longitudinaux, les deux autres recouvrant la couche adhésive le long du bord proximal 13 et du bord distal 14.

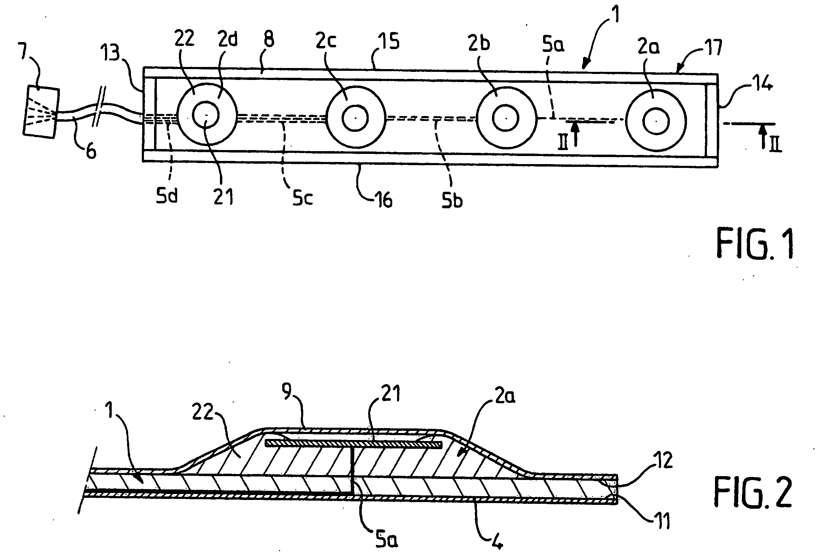

Les électrodes sont entièrement recouvertes d'un film de protection 9, représenté uniquement sur la figure 2 pour des raisons de clarté, destiné à être retiré avant utilisation des électrodes. Ce film de protection 9 et la pellicule pelable 8 sont disposés bord à bord. Dans une variante de réalisation, le film de protection 9 vient recouvrir ladite pellicule pelable, le retrait du film de protection pouvant alors permettre de retirer également la pellicule pelable. Dans une autre variante de réalisation, le film de protection constitue également la pellicule pelable qui recouvre la couche adhésive 17. Il pourrait également être prévu de recouvrir chaque électrode d'un film de protection individuel.

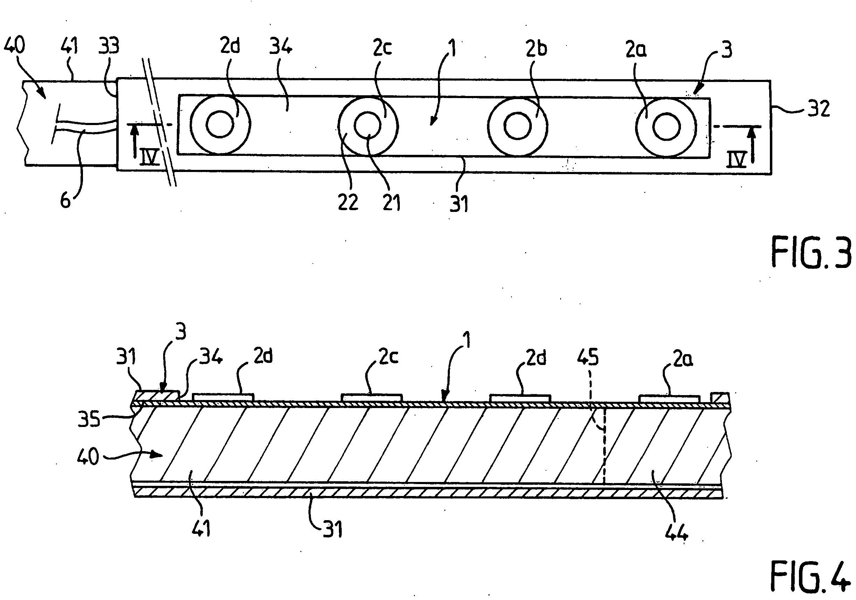

En référence aux figures 3 et 4, la housse de protection 3 est formée d'une paroi tubulaire 31 en un matériau plastique flexible isolant, par exemple en latex ou en polyuréthane, présentant une extrémité distale fermée 32, et une extrémité proximale 33 ouverte par laqu elle la housse peut être enfilée sur l'extrémité distale d'un endoscope. La longueur de la housse est prévue pour recouvrir toute la partie de l'endoscope qui sera introduite par la bouche du patient. Selon l'invention, la paroi tubulaire 31 de la housse comprend une ouverture longitudinale 34, rectangulaire, traversant la paroi tubulaire de part en part. La longueur de l'ouverture est inférieure à la longueur de la bande et au moins égale à la longueur de bande support sur laquelle sont agencées les électrodes, et de préférence, inférieure ou sensiblement égale à la longueur séparant les deux portions transversales de la couche adhésive 17. La largeur de l'ouverture est quant à elle inférieure à la largeur de la bande support et sensiblement égale ou supérieure au diamètre des membranes conductrices des électrodes. Pour assurer un contact optimal entre les électrodes et le tissu auriculaire au moment de la cardioversion la largeur de l'ouverture 34 est de préférence inférieur ou sensiblement égale à la longueur séparant les deux portions longitudinales de la couche adhésive 17, et sensiblement égale ou supérieure au diamètre des membranes protectrices 22 non conductrices des électrodes. A titre d'exemple, la largeur de l'ouverture est sensiblement égale au diamètre des membranes protectrices 22, tel qu'illustré sur la figure 3.

La bande support et la housse sont destinées à être positionnées sur l'extrémité distale 41 d'un endoscope tubulaire flexible 40, de structure connue en soi, relié à son extrémité proximale 42 à une poignée de commande 43 tel qu'illustré à la figure 4. Avantageusement, la bande support et la housse peuvent être utilisées avec un endoscope comportant à son extrémité distale 41 un capteur multiplan à ultrasons pour permettre de réaliser à la fois une échographie et une cardioversion. Le capteur multiplan (non représenté) est positionné sur une portion distale 44 articulée par rapport au reste de l'endoscope. Cette articulation est représentée schématiquement par la référence 45 sur la figure 4. De manière connue, la portion articulée 44 peut être manoeuvrée mécaniquement en flexion par l'intermédiaire d'un guide de commande (non représenté) coaxialement logé dans le tube endoscopique par deux molettes 47, 48 prévues sur la poignée, chacune équipée d'un frein. La poignée est munie d'une autre molette 49 manuelle permettant la commande en rotation du plan de coupe du capteur à ultrasons qui est mécaniquement dirigé annulairement.

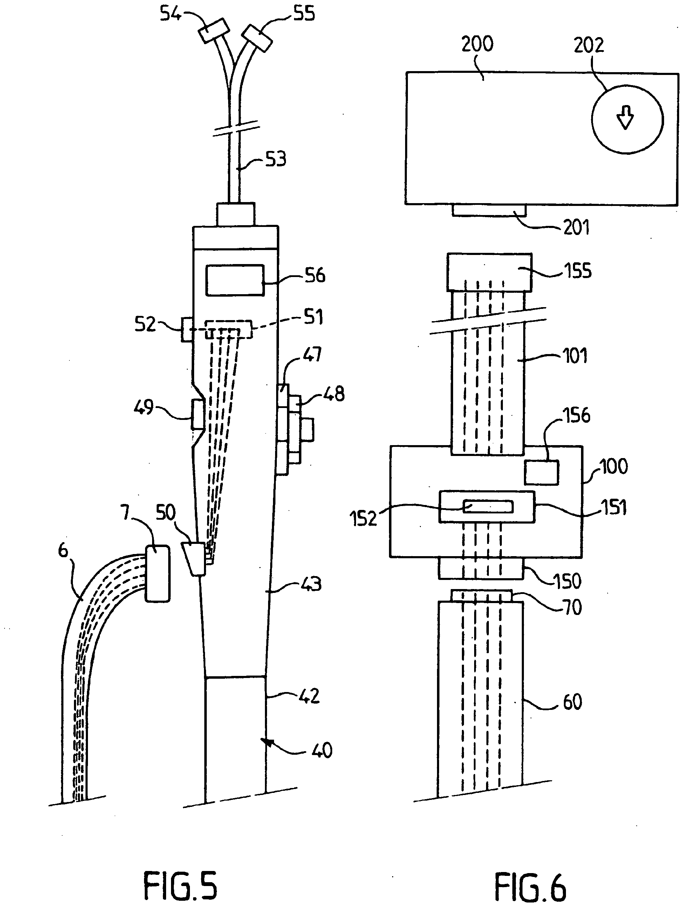

La bande support et la housse de protection peuvent être utilisées de la manière suivante. La pellicule pelable 4 est retirée de la face interne à surface adhésive et la bande support est fixée par cette face sur l'extrémité distale 41 de l'endoscope, de sorte que l'électrode 2a, la plus proche du bord distal 14 de la bande support, soit fixée sur la portion distale articulée 44 de l'endoscope, entre le capteur et l'articulation 45, et les trois autres électrodes 2b, 2c et 2d soient positionnées sur la portion non articulée 44 de l'extrémité distale 41 de l'endoscope au voisinage de l'articulation. Les fils de conduction sont alors disposés entre la paroi tubulaire de l'endoscope et la bande support. La housse est ensuite emmanchée sur l'extrémité distale de l'endoscope. Les quatre portions de la pellicule pelable 8 et le film de protection 9 sont retirés et la housse est fixée sur la bande support par collage au moyen de la couche adhésive contre la face interne 35 de la paroi tubulaire de la housse, autour de l'ouverture 34, en appliquant manuellement une pression sur la face externe de la paroi tubulaire de la housse. Les figures 4 et 5 illustrent le système selon l'invention après fixation sur l'extrémité distale de l'endoscope. Alternativement, la housse et la bande support peuvent être assemblées l'une à l'autre après avoir retiré la pellicule pelable 8 et le film de protection 9, cet assemblage est alors emmanché sur l'extrémité distale de l'endoscope et la bande est collée sur l'endoscope. Dans ce cas, la pellicule pelable 4 peut être enlevée avant ou après le positionnement dudit assemblage sur l'endoscope. Pour faciliter cette opération d'enlèvement de la pellicule pelable 4, il peut être prévu une pellicule pelable munie d'une languette, partant du bord distal 14 de la bande support et s'étendant sur une longueur telle que son extrémité libre soit accessible de l'extérieur lorsque la bande support est fixée à la housse de protection.

A titre d'exemple, bien que non représenté sur les figures, la bande mesure de 15 à 20 centimètres et les quatres électrodes sont agencées sur une longueur d'environ 12 à 15 centimètres. Chaque électrode présente un diamètre de l'ordre de 1,5 à 2 centimètres. La distance entre l'électrode distale extrême 2a et l'électrode 2b suivante est d'environ 2 à 2,5 centimètres, alors que les électrodes restantes sont écartées de 1 à 2 centimètres seulement. La bande est fixée sur l'endoscope de sorte que l'électrode distale extrême soit placée à une distance d'environ 3,5 à 4,5 centimètres du sommet distal de l'endoscope dans sa portion articulée 44.

Les électrodes peuvent ainsi être positionnées de manière précise à l'endroit désiré sur l'endoscope. Par ailleurs, la housse est fixée par l'intermédiaire de la bande support à l'endoscope, ce qui assure un bon maintien de la housse sur l'endoscope et facilite la manipulation de l'ensemble, en particulier lors de l'extraction de l'endoscope hors de l'oesophage. Il est souhaitable que le collage de la bande support à la housse assure une étanchéité au moins vis-à- vis des liquides et sucs présents dans l'oesophage. Un tel collage étanche de la bande support à la housse permet d'éviter toute contamination de l'endoscope lors de son utilisation et ainsi de simplifier la procédure de lavage de celui- ci entre deux opérations. Pour assurer un collage correct de la housse sur la bande support portant les électrodes, la bande support peut comprendre un repère, tel qu'un trait transversal de couleur rouge, situé sur la face externe 12 de la bande support entre l'électrode distale extrême 2a destinée à être fixée sur la portion distale articulée d'un endoscope et l'électrode suivante 2b destinée à être fixée sur la portion non articulée 44 de l'extrémité distale de l'endoscope. La distance qui sépare ce trait de repère de l'électrode distale extrême 2a sera définie en fonction des différents types d'endoscope disponibles dans le commerce. De cette manière, l'opérateur commence par fixer la bande support au niveau de la ligne d'articulation 45 de l'endoscope à l'aide de ce repère et fixe ensuite le reste de la bande support. Ainsi, la housse sera parfaitement collée sur la bande support et l'étanchéité sera respectée. Dans une variante de réalisation, il pourrait être prévu une couche adhésive sur la face interne 35 de la paroi tubulaire de la housse, entourant l'ouvert ure et recouverte d'une pellicule pelable, pour remplacer ou compléter la couche adhésive 17 prévue sur la face externe de la bande support.

Lorsque la cardioversion a été réalisée, la housse et la bande support peuvent être retirées de l'extrémité distale de l'endoscope. L'adhérence de la bande support à l'endoscope et l'adhérence de la bande support à la housse peuvent être déterminées de sorte que lorsque l'opérateur retire la housse, la bande support reste fixée à l'endoscope, ou de sorte que la bande support reste fixée à la housse de protection lors du retrait de la housse par l'opérateur. Cette dernière solution est préférée car elle permet de limiter au maximum les risques de contamination de l'endoscope.

Dans le cas d'une utilisation de la housse pour un endoscope pour échographie muni d'un capteur à ultrasons, un gel, destiné à recouvrir le capteur à ultrasons pour obtenir une visualisation correcte à travers la housse lors de l'échographie, peut être placé à l'extrémité distale fermée 32. Un système rigide de fermeture à clip (non représenté) est agencé sur l'extrémité distale de l'endoscope et vient pincer la paroi de la housse entre l'extrémité distale 32 et l'ouverture 34, par exemple à 2-3 centimètres de l'extrémité distale, pour retenir le gel dans la housse. Après avoir retiré manuellement le système de fermeture, l'extrémité distale de l'endoscope est insérée dans la housse, et le capteur à ultrasons est recouvert de manière automatique par le gel. La présence de ce gel in situ permet d'éviter l'étape d'adjonction de gel par le médecin, au moyen d'une seringue insérée par l'ouverture proximale ouverte de la housse.

La gaine 6 de longueur appropriée est disposée le long de l'endoscope et peut être assemblée à celui -ci par tout moyen approprié. Dans une première variante illustrée à la figure 5, le système selon l'invention comprend un moyen de commande de cardioversion intégré à la poignée de commande 42 de l'endoscope et auquel le connecteur 7 de la gaine 6 est connecté. La poignée de commande comprend une fiche réceptrice 50 pour permettre la connexion du connecteur à un module 51 intégré dans la poignée. Ce module servant à charger l'énergie sélectionnée et à réaliser la cardioversion est commandé par un bouton de commande externe 52. Un câble 53 permet de relier la poignée d'une part à un appareil d'échographie et d'autre part à un appareil de cardioversion. L'extrémité du câble 53 bifurque en deux connecteurs, un premier connecteur 54 qui permet de relier le capteur à ultrasons à un appareil d'échographie et un deuxième connecteur 55 qui permet de relier le module 51 à un appareil de cardioversion. La poignée de commande 52 comporte en outre un écran numérique de visualisation 56 pour indiquer le nombre de Joules de la charge électrique à déclencher, ainsi qu'un voyant lumineux muni d'un avertisseur sonore pour indiquer la fin de la charge d'énergie.

Dans une autre variante de réalisation illustrée à la figure 6, le moyen de commande de la cardioversion est formé par un boîtier 100 indépendant de la poignée, mais apte à être assemblé sur celle-ci. Le boîtier 100 comprend un module de commande 151, commandé par un bouton 152 et intercalé entre une fiche réceptrice 150 permettant de connecter au module 151 le connecteur 70 d'une gaine 60 contenant les fils de conduction des électrodes et un câble de sortie 101 pourvu à son extrémité d'un connecteur 155 permettant la connexion à un appareil de cardioversion 200 par l'intermédiaire d'une fiche réceptrice complémentaire 201. De manière connue, l'appareil de cardioversion comprend un condensateur apte à délivrer la charge sélectionnée manuellement par un commutateur externe 202. Le boîtier 100 comprend des moyens d'assemblage (non représentés) permettant d'assembler le boîtier sur la poignée d'un endosc ope. Le boîtier comprend en outre un écran numérique de visualisation 156 pour indiquer le nombre de Joules de la charge électrique à déclencher, qui a été sélectionné par le commutateur 202 de l'appareil de cardioversion, ainsi qu'un voyant lumineux muni d'un avertisseur sonore pour indiquer la fin de la charge d'énergie.

Les deux moyens de commande décrits ci-dessus s'utilisent de la manière suivante. Après avoir sélectionné la charge d'énergie électrique appropriée, l'opérateur commande par une brève pression sur le bouton de commande la mise en charge du condensateur de l'appareil de cardioversion sous le contrôle du voyant muni de l'avertisseur sonore qui indique la fin de la charge choisie. Une fois la charge effectuée, une seconde pression sur le même bouton permet de déclencher la décharge par les électrodes pour le rétablissement du rythme sinusal. Avec ce moyen de commande intégré à la poignée ou assemblé à la poignée, l'opérateur peut gérer la réalisation de la cardioversion sans l'aide d'une tierce personne. Le moyen de commande indépendant décrit ci- dessus permet d'adapter le système à électrode selon l'invention à tout type d'endoscope existant sur le marché.

Bien que l'invention ait été décrite en liaison avec deux modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention. Le système selon l'invention comprenant la bande support et la housse de protection peut être utilisé avec tout type d'endoscope, qu'il soit destiné à une échographie ou non. Comme décrit précédemment, les électrodes peuvent être placées sur la portion distale articulée d'un endoscope, que celle-ci s oit articulée manuellement ou par l'utilisation d'un matériau à mémoire de forme par exemple. Bien entendu, la longueur de l'ouverture pourra être adaptée en fonction du nombre d'électrodes portées par la bande support qui peut varier, de préférence de 3 à 6, et les dimensions de l'ouverture de la housse pourront bien entendu être adaptées au nombre et au type d'électrodes.

## Revendications

1. Système à électrode prêt-à-monter destiné à être utilisé avec un endoscope pour effectuer une cardioversion par voie oesophagienne, comportant une housse de protection (3) de forme générale tubulaire présentant une extrémité distale fermée (32) et une extrémité proximale ouverte (33), ladite housse étant destinée à être emmanchée sur l'endoscope (40) pour recouvrir au moins l'extrémité distale (41) de l'endoscope, au moins une électrode (2a-d) comprenant une membrane électrique conductrice (21) connectée à un fil de conduction électrique (5a-d) destiné à être relié par son extrémité libre à un appareil de cardioversion, des premiers moyens de fixation aptes à permettre la fixation de ladite au moins une électrode sur l'extrémité distale (41) de l'endoscope, et des seconds moyens de fixation (17) aptes à permettre la fixation de ladite au moins une électrode sur la paroi tubulaire (31) de la housse, de façon que ladite membrane conductrice soit directement accessible de l'extérieur de la housse, **caractérisé par le fait qu'**il comprend au moins une bande support (1) apte à être fixée par une première face dite interne (11) sur l'endoscope par lesdits premiers moyens de fixation et portant sur sa seconde face dite externe (12) ladite au moins une électrode (2a-d), la paroi tubulaire (31) de ladite housse de protection (3) étant munie d'au moins une ouverture (34) de dimensions inférieures à celles de la bande support, les seconds moyens de fixation (17) étant prévus sur la housse de protection et/ou la bande support pour permettre une fixation de la bande support par sa seconde face externe (12) contre la face interne (35) de la paroi tubulaire de la housse de protection, de sorte que ladite au moins une ouverture soit fermée et que la membrane conductrice de ladite électrode soit accessible de l'extérieur à travers ladite ouverture.

2. Système selon la revendication 1, **caractérisé par le fait que** la largeur de l'ouverture (34) de la housse de protection est au moins égale à la dimension transversale de la membrane conductrice (21) de l'électrode (2a-d), de sorte que ladite membrane conductrice soit logée à travers ladite ouverture lorsque la bande support (1) est fixée à la housse de protection par les seconds moyens de fixation (17).

3. Système selon la revendication 1 ou 2, **caractérisé par le fait que** la surface de la première face interne (11) de la bande support (1) comprend une première couche auto-adhésive qui constitue lesdits premiers moyens de fixation.

4. Système selon l'une des revendications 1 à 3, **caractérisé par le fait que** les seconds moyens de fixation sont constitués par une deuxième couche auto-adhésive (17) située à la périphérie de l'ouverture (34) sur la face interne (35) de la paroi tubulaire (31) de la housse de protection (3) et/ou sur la périphérie (13-16) de la seconde face externe (12) de la bande support (1).

5. Système selon la revendication 4, **caractérisé par le fait que** chaque couche auto-adhésive est recouverte d'une pellicule pelable (4) destinée à être retirée avant la fixation de la bande support (1).

6. Système selon l'une des revendications 1 à 5, **caractérisé par le fait qu'**il comprend de 3 à 6 électrodes (2a-d) espacées longitudinalement sur la bande support (1), la paroi tubulaire (31) de la housse de protection étant munie d'une ouverture longitudinale (34) de longueur correspondante.

7. Système selon l'une des revendications 1 à 6, **caractérisé par le fait que** le ou les fils de conduction (5a-d) sont portés par la bande support (1), s'étendent jusqu'au bord transversal proximal (13) de la bande support et sont logés à partir de ce bord transversal proximal (13) dans une gaine isolante (6) reliée à son extrémité opposée à un connecteur (6, 60) permettant la connexion du ou des fils de conduction à l'appareil de cardioversion.

8. Système selon l'une des revendications 1 à 7, **caractérisé par le fait que** la ou les électrodes (2a-d) sont recouvertes par au moins un film de protection ((9) apte à être retiré avant l'utilisation de la ou des électrodes.

9. Système selon l'une des revendications 1 à 8, **caractérisé par le fait qu'**il comporte un moyen de commande externe comprenant un boîtier (100) muni de moyens de montage pour permettre le montage dudit boîtier sur l'extrémité proximale de l'endoscope, d'un premier moyen de connexion externe (150), pour connecter le ou les fils de conduction (5a-d) de la ou des électrodes (2a-d), d'un deuxième moyen de connexion (101, 155) pour connecter le boîtier à l'appareil de cardioversion (200), et d'un module (151) pour la charge de l'énergie électrique destinée à produire les chocs électriques et pour le déclenchement de ces chocs, ledit module étant commandé par un organe de commande externe (152) et étant relié aux premier et second moyens de connexion.

10. Ensemble du système selon l'une des revendications 1 à 8 et d'un endoscope (40) muni à son extrémité proximale (42) d'une poignée de commande (43), destiné à être utilisé pour effectuer une cardioversion par voie oesophagienne, **caractérisé par le fait que** la housse de protection (3) est emmanchée sur l'endoscope (40) et recouvre au moins l'extrémité distale (41) de l'endoscope, ladite au moins une électrode (2a-d) est fixée d'une part sur l'extrémité distale (41) de l'endoscope par les premiers moyens de fixation et, d'autre part, sur la paroi tubulaire de la housse par les seconds moyens de fixation (17) de façon que ladite membrane conductrice soit directement accessible de l'extérieur de la housse, ledit fil de conduction (5a-d) étant relié par son extrémité libre à un moyen de connexion externe (50, 150) situé au niveau de ladite poignée de commande.

11. Ensemble selon la revendication 10 **caractérisé par le fait que** la poignée de commande (43) intègre un moyen de commande interne de cardioversion, ledit système comprenant un module (51) pour la charge de l'énergie électrique destinée à produire les chocs électriques et pour le déclenchement de ces chocs, ledit module étant commandé par un organe externe de commande (52) et étant relié audit premier moyen de connexion externe (50) pour connecter la ou les fils de conduction (5a-d) de la ou des électrodes, et à un deuxième moyen de connexion (55) pour le connecter à l'appareil de cardioversion.

12. Ensemble selon la revendication 10 **caractérisé par le fait qu'**il comprend un moyen de commande externe comprenant un boîtier (100) muni de moyens de montage pour permettre le montage dudit boîtier sur l'extrémité proximale (42) de l'endoscope, dudit premier moyen de connexion externe (150) pour connecter le ou les fils de conduction (5a-d) de la ou des électrodes (2a-d) au boîtier, d'un deuxième moyen de connexion (101, 155) pour connecter le boîtier à l'appareil de cardioversion (200), et d'un module (151) pour la charge de l'énergie électrique destinée à produire les chocs électriques et pour le déclenchement de ces chocs, ledit module étant commandé par un organe de commande externe (152) et étant relié aux premier et second moyens de connexion.

13. Ensemble selon l'une des revendications 10 à 12, **caractérisé par le fait qu'**une portion distale (41) de l'endoscope est munie d'un capteur à ultrasons de manière à former un dispositif permettant d'effectuer à la fois une échographie et une cardioversion par voie oesophagienne.

14. Ensemble selon la revendication 13, **caractérisé par le fait que** la portion distale (44) de l'endoscope (40) qui comporte le capteur est articulée par rapport au reste de l'endoscope, au moins une électrode (2a) étant fixée de part et d'autre de l'articulation.

## Claims

1. A ready-to-install electrode system designed for use with an endoscope for performing cardioversion through the esophagus, comprising a globally tubular protective cover (3) having a closed distal end (32) and an open proximal end (33), said cover being designed to be fitted on the endoscope (40) to cover at least the distal end (41) of the endoscope, at least one electrode (2a-d) including a conductive electric membrane (21) connected to an electric conduction wire (5a-d) designed to be connected by its free end to a cardioversion apparatus, first fastening means that allow said at least one electrode to be fastened to the distal end (41) of the endoscope, and second fastening means (17) that allow said at least one electrode to be fastened on the tubular wall (31) of the cover, such that the conductive membrane is directly accessible from outside the cover, **characterized in that** it comprises at least one support strip (1) that can be fastened by a first so-called inside face (11) on the endoscope by said first fastening means, and carrying, on its second so-called outer face (12), said at least one electrode (2a-d), the tubular wall (31) of said protective cover (3) being provided with at least one opening (34) with dimensions smaller than those of the support strip, the second fastening means (17) being provided on the protective cover and/ or the support strip to allow the support strip to be fastened by its second outside face (12) against the inside face (35) of the tubular wall of the protective cover, such that at least one opening is closed and the conductive membrane of said electrode is accessible from the outside through said opening.

2. The system according to claim 1, **characterized in that** the width of the opening (34) of the protective cover is at least equal to the crosswise dimension of the conductive membrane (21) of the electrode (2a-d), such that said conductive membrane is housed through said opening when the support strip (1) is fastened to the protective cover by the second fastening means (17).

3. The system according to claim 1 or 2, **characterized in that** the surface of the first inside face (11) of the support strip (1) comprises a first self-adhesive layer that constitutes said first fastening means.

4. The system according to one of claims 1 to 3, **characterized in that** the second fastening means are made up of a second self-adhesive layer (17) situated on the periphery of the opening (34) on the inside face (35) of the tubular wall (31) of the protective cover (3) and/or on the periphery (13-16) of the second outside face (12) of the support strip (1).

5. The system according to claim 4, **characterized in that** each self-adhesive layer is covered by a peelable film (4) designed to be removed before the support strip (1) is fastened.

6. The system according to one of claims 1 to 5, **characterized in that** it comprises from 3 to 6 electrodes (2a-d) spaced longitudinally on the support strip (1), the tubular wall (31) of the protective cover being provided with a longitudinal opening (34) of corresponding length.

7. The system according to one of claims 1 to 6, **characterized in that** the conductive wire(s) (5a-d) are borne by the support strip (1), extend up to the proximal transverse edge (13) of the support strip, and are housed from that proximal transverse strip (13) in an insulating sheath (6) connected at its opposite end to a connector (6, 60) allowing the connection of the conductive wire(s) to the cardioversion device.

8. The system according to one of claims 1 to 7, **characterized in that** the electrode(s) (2a-d) are covered by at least one protective film (9) that can be removed before use of the electrode(s).

9. The system according to one of claims 1 to 8, **characterized in that** it comprises an external control means including a box (100) equipped with mounting means to allow the mounting of said box on the proximal end of the endoscope, a first external connecting means (150), to connect the conductive wire(s) (5a-d) of the electrode(s) (2a-d), a second connecting means (101, 155) for connecting the box to the cardioversion apparatus (200), and a module (151) for the electric power charge designed to produce electric shocks and for triggering those shocks, said module being controlled by an external control element (152) and being connected to the first and second connecting means.

10. An assembly of the system according to one of claims 1 to 8 and an endoscope (40) equipped at its proximal end (42) with a control handle (43) designed to be used to carry out a cardioversion esophageally, **characterized in that** the protective cover (3) is fitted on the endoscope (40) and covers at least the distal end (41) of the endoscope, said at least one electrode (2a-d) is fastened, on the one hand, on the distal end (41) of the endoscope by the first fastening means and, on the other hand, on the tubular wall of the cover by the second fastening means (17) such that said conductive membrane is directly accessible from the outside of the cover, said conductive wire (5a-d) being connected by its free end to an external connecting means (50, 150) located at the level of said control handle.

11. The assembly according to claim 10, **characterized in that** the control handle (43) integrates an internal cardioversion control means, said system comprising a module (51) for the electric power charge intended to produce the electrical shocks and for triggering those shocks, said module being controlled by an external control element (52) and being connected to said first external connecting means (50) to connect the conductive wire(s) (5a-d) of the electrode(s), and a second connecting means (55) for connecting it to the cardioversion apparatus.

12. The assembly according to claim 10, **characterized in that** it comprises an external control means comprising a box (100) equipped with mounting means for allowing the mounting of said box on the proximal end (42) of the endoscope, said first external connecting means (150) to connect the conductive wire(s) (5a-d) of the electrode(s) (2a-d) to the box, a second connecting means (101, 155) for connecting the box to the cardioversion device (200), and a module (151) for the electric power charge designed to produce electric shocks and for triggering those shocks, said module being controlled by an external control element (152) and being connected to the first and second connecting means.

13. The assembly according to one of claims 10 to 12, **characterized in that** a distal portion (41) of the endoscope is provided with an ultrasonic sensor so as to form a device that allows both echography and esophageal cardioversion to be carried out.

14. The assembly according to claim 13, **characterized in that** the distal portion (44) of the endoscope (40) that comprises the sensor is articulated relative to the rest of the endoscope, at least one electrode (2a) being fastened on both sides of the articulation.

## Patentansprüche

1. Elektrodensystem-Bausatz, dazu vorgesehen, mit einem Endoskop verwendet zu werden, um eine Kardioversion auf ösophagealem Weg durchzuführen, umfassend eine Schutzhülle (3) mit einer im Allgemeinen rohrförmigen Gestalt, aufweisend ein geschlossenes distales Ende (32) und ein offenes proximales Ende (33), wobei die Hülle dazu vorgesehen ist, auf das Endoskop (40) gepresst zu werden, um mindestens das distale Ende (41) des Endoskops abzudecken, wobei mindestens eine Elektrode (2a-d) eine leitende elektrische Membran (21) umfasst, die mit einem elektrischen Leitungsdraht (5a-d) verbunden ist, der dazu vorgesehen ist, mit seinem freien Ende mit einem Kardioversionsgerät verbunden zu sein, erste Befestigungsmittel, die dazu geeignet sind, die Befestigung der mindestens einen Elektrode auf dem distalen Ende (41) des Endoskops zu ermöglichen, und zweite Befestigungsmittel (17), die dazu geeignet sind, die Befestigung der mindestens einen Elektrode auf der rohrförmigen Wand (31) der Hülle zu ermöglichen, so dass die leitende Membran direkt von der Außenseite der Hülle zugänglich ist, **dadurch gekennzeichnet, dass** es mindestens ein Trägerband (1) umfasst, das dazu geeignet ist, mit einer ersten Seite, genannt Innenseite (11) durch die ersten Befestigungsmittel auf dem Endoskop befestigt zu sein und auf seiner zweiten Seite, genannt Außenseite (12), die mindestens eine Elektrode (2a-d) trägt, wobei die rohrförmige Wand (31) der Schutzhülle (3) mit mindestens einer Öffnung (34) mit Abmessungen ausgestattet ist, die kleiner als diejenigen des Trägerbands sind, wobei die zweiten Befestigungsmittel (17) auf der Schutzhülle und/oder dem Trägerband vorgesehen sind, um eine Befestigung des Trägerbandes mit seiner zweiten Außenseite (12) gegen die Innenseite (35) der rohrförmigen Wand der Schutzhülle zu ermöglichen, so dass die mindestens eine Öffnung geschlossen ist, und dass die leitende Membran der Elektrode von außen über die Öffnung zugänglich ist.,

2. System nach Anspruch 1, **dadurch gekennzeichnet, dass** die Breite der Öffnung (34) der Schutzhülle mindestens gleich der Querabmessung der leitenden Membran (21) der Elektrode (2a-d) ist, so dass die leitende Membran durch die Öffnung angebracht wird, wenn das Trägerband (1) durch die zweiten Befestigungsmittel (17) an die Schutzhülle befestigt ist.

3. System nach Anspruch 1 oder 2, **dadurch gekennzeichnet, dass** die Oberfläche der ersten Innenseite (11) des Trägerbands (1) eine erste selbsthaftende Schicht umfasst, die die ersten Befestigungsmittel darstellt.

4. System nach einem der Ansprüche 1 bis 3, **dadurch gekennzeichnet, dass** die zweiten Befestigungsmittel aus einer zweiten selbsthaftenden Schicht (17) bestehen, die sich auf dem Umfang der Öffnung (34) auf der Innenseite (35) der rohrförmigen Wand (31) der Schutzhülle (3) und/oder auf dem Umfang (13-16) der zweiten Außenseite (12) des Trägerbands (1) befindet.

5. System nach Anspruch 4, **dadurch gekennzeichnet, dass** jede selbsthaftende Schicht mit einer abziehbaren Folie (4) bedeckt ist, die dazu vorgesehen ist, vor der Befestigung des Trägerbandes (1) entfernt zu werden.

6. System nach einem der Ansprüche 1 bis 5, **dadurch gekennzeichnet, dass** es 3 bis 6 Elektroden (2a-d) umfasst, die der Länge nach auf dem Trägerband (1) beabstandet sind, wobei die rohrförmige Wand (31) der Schutzhülle mit einer Längsöffnung (34) mit entsprechender Länge ausgestattet ist.

7. System nach einem der Ansprüche 1 bis 6, **dadurch gekennzeichnet, dass** der Leitungsdraht oder die Leitungsdrähte (5a-d) vom Trägerand (1) getragen werden, die sich bis zum proximalen Querrand (13) des Trägerbandes erstrecken und ab diesem proximalen Querrand (13) in einem isolierenden Mantel (6) aufgenommen sind, der an seinem gegenüber liegenden Ende mit einem Verbindungsglied (6, 60) verbunden ist, das die Verbindung des Leitungsdrahts oder der Leitungsdrähte mit dem Kardioversionsgerät ermöglicht

8. System nach einem der Ansprüche 1 bis 7, **dadurch gekennzeichnet, dass** die Elektrode(n) (2a-d) von mindestens einem Schutzfilm (9) bedeckt sind, der vor der Verwendung der Elektrode(n) entfernt werden kann.

9. System nach einem der Ansprüche 1 bis 8, **dadurch gekennzeichnet, dass** es ein Mittel zur externen Steuerung aufweist, umfassend ein Gehäuse (100), das mit Befestigungsmitteln versehen ist, um die Befestigung des Gehäuses auf dem proximalen Ende des Endoskops zu ermöglichen, mit einem ersten externen Verbindungsmittel (150), um den Leitungsdraht oder die Leitungsdrähte (5a-d) der Elektrode(n) (2a-d) zu verbinden, mit einem zweiten Verbindungsmittel (101, 155), um das Gehäuse mit dem Kardioversionsgerät (200) zu verbinden, und mit einem Modul (151) für die Ladung der elektrischen Energie, die dazu vorgesehen ist, die elektrische Schocks herzustellen und für die Auslösung dieser Schocks, wobei das Modul von einem externen Steuerorgan (152) gesteuert wird und mit dem ersten und dem zweiten Verbindungsmittel verbunden ist.

10. Einheit des Systems nach einem der Ansprüche 1 bis 8 und eines Endoskops (40), ausgestattet an ihrem proximalen Ende (42) mit einem Steuergriff (43) der dazu vorgesehen ist, verwendet zu werden, um eine Kardioversion auf ösophagealem Weg durchzuführen, **dadurch gekennzeichnet, dass** die Schutzhülle (3) auf das Endoskop (40) gepresst ist und mindestens das distale Ende (41) des Endoskops abdeckt, wobei die mindestens eine Elektrode (2a-d) einerseits auf dem distalen Ende (41) des Endoskops durch die ersten Befestigungsmittel und andererseits auf der rohrförmigen Wand der Hülle durch die zweiten Befestigungsmittel (17) befestigt ist, so dass die leitende Membran direkt von der Außenseite der Hülle zugänglich ist, wobei der Leitungsdraht (5a-d) mit seinem freien Ende mit einem externen Verbindungsmittel (50, 150) verbunden ist, das sich auf der Ebene des Steuergriffs befindet.

11. Einheit nach Anspruch 10, **dadurch gekennzeichnet, dass** der Steuergriff (43) ein Mittel zur internen Steuerung der Kardioversion einschließt, wobei das System ein Modul (51) für die Ladung der elektrischen Energie umfasst, die dazu vorgesehen ist, die elektrischen Schocks zu erzeugen und für die Auslösung dieser Schocks, wobei das Modul von einem externen Steuerorgan (52) gesteuert wird und mit dem ersten externen Verbindungsmittel (50) verbunden ist, um den Leitungsdraht oder die Leitungsdrähte (5a-d) der Elektrode(n) zu verbinden, und mit einem zweiten Verbindungsmittel (55), um es mit dem Kardioversionsgerät zu verbinden.

12. Einheit nach Anspruch 10, **dadurch gekennzeichnet, dass** es ein externes Steuermittel aufweist, umfassend ein Gehäuse (100), das mit Befestigungsmitteln versehen ist, um die Befestigung des Gehäuses auf das proximale Ende (42) des Endoskops zu ermöglichen, mit dem ersten externen Verbindungsmittel (150), um den Leitungsdraht oder die Leitungsdrähte (5a-d) der Elektroden (2a-d) mit dem Gehäuse zu verbinden, mit einem zweiten Verbindungsmittel (101, 155), um das Gehäuse mit dem Kardioversionsgerät (200) zu verbinden, und mit einem Modul (151) für die Ladung der elektrischen Energie, die dazu vorgesehen ist, die elektrischen Schocks herzustellen, und für die Auslösung dieser Schocks, wobei das Modul von einem externen Steuerorgan (152) gesteuert wird und mit dem ersten und dem zweiten Verbindungsmittel verbunden ist.

13. Einheit nach einem der Ansprüche 10 bis 12, **dadurch gekennzeichnet, dass** ein distaler Abschnitt (41) des Endoskops mit einem Ultraschallsensor ausgestattet ist, um eine Vorrichtung zu bilden, die es ermöglicht, gleichzeitig eine Echographie und eine Kardioversion auf ösophagealem Weg durchzuführen.

14. Einheit nach Anspruch 13, **dadurch gekennzeichnet, dass** der distale Abschnitt (44) des Endoskops (40), der den Sensor umfasst, mit Bezug auf das restliche Endoskop gelenkig verbunden ist, wobei mindestens eine Elektrode (2a) auf beiden Seiten der gelenkigen Verbindung befestigt ist.
